# EUROPEAN PATENT SPECIFICATION

(11) **EP 2 977 460 B1**
(45) Date of publication and mention of the grant of the patent: **08.05.2019**
(21) Application number: 14767484.0
(22) Date of filing: 20.03.2014
(51) Int. Cl.: C12P 19/04, C08B 37/08, A61K 31/728, C12R 1/46, A61L 27/58, A61L 31/14, C12P 19/26, C08L 5/08, A61L 27/20, A61L 31/04

(54) **PREPARATION METHOD FOR HYALURONIC ACID, AND ANTI-ADHESIVE COMPOSITION COMPRISING HYALURONIC ACID PREPARED BY SAME PREPARATION METHOD**
HERSTELLUNGSVERFAHREN FÜR HYALURONSÄURE UND HAFTSCHUTZZUSAMMENSETZUNG MIT NACH DEM HERSTELLUNGSVERFAHREN HERGESTELLTE HYALURONSÄURE
PROCÉDÉ DE PRÉPARATION D'ACIDE HYALURONIQUE, ET COMPOSITION ANTIADHÉSIVE COMPRENANT DE L'ACIDE HYALURONIQUE PRÉPARÉ PAR LE MÊME PROCÉDÉ DE PRÉPARATION

(30) Priority: 20.03.2013 KR 20130029876
(43) Date of publication of application: 27.01.2016
(73) Proprietor: Ildong Pharm Co., Ltd., Seoul 137-733 (KR)
(72) Inventor: KANG, Dae-Jung, Giheung-gu Yongin-si Gyeonggi-do 446-582 (KR); KIM, Tae-Yoon, Hwaseong-si Gyeonggi-do 445-320 (KR); KANG, Jae-Hoon, Seocho-gu Seoul 137-923 (KR)
(74) Representative: Stolmár & Partner Patentanwälte PartG mbB
(86) International application number: PCT/KR2014/002364
(87) International publication number: WO 2014/148847

(56) References cited:
- EP-A2- 2 371 371
- WO-A1-2010/125069
- WO-A1-2012/057381
- JP-A- H10 113 197
- KR-A- 20100 048 778
- KR-A- 20100 061 963
- KR-A- 20130 025 337
- KR-A- 20130 035 808
- US-A1- 2012 232 261

## Description

### [Technical Field]

The present invention relates to a preparation method for hyaluronic acid possessing a low degradation rate in the body, and an anti-adhesive composition comprising hyaluronic acid prepared by said preparation method. More specifically, the present invention relates to a preparation method for hyaluronic acid possessing a low degradation rate in the body, comprising a step of culturing *Streptococcus dysgalactiae strain* ID9103 (KCTC11818BP) in a medium comprising a carbon source and a nitrogen source, and an anti-adhesive composition comprising hyaluronic acid prepared by said preparation method.

### [Background Art]

Hyaluronic acid ((HA), Hyaluronan, (C₁₄H₂₀NNaO₁₁)n (n>1000)) is a polymer existing in living organisms, and is a polysaccharide, called glycosaminoglycan. It has a structure which is composed of alternating D-glucuronic acid and N-acetylglucosamine, linked together via alternating *β*-1,3 and *β*-1,4 glycosidic bonds. It is a water-soluble material with significantly high viscosity and elasticity, while its molecular weight ranges variably from 1,000 to 10,000,000Da (daltons) with its extensive structure of straight chain.

Hyaluronic acid possesses a high efficacy and effectiveness as a lubricant in a physical friction state due to its high moisturizing effect. Also, it has preferable advantages in various effects and properties such as protection against bacterial invasion, leading to its usefulness for a wide range of indications. In order to develop hyaluronic acid, a biological tissue extraction method or a microorganism culturing method has been used basically. However, since a chicken comb extraction method causes many disadvantages such as virus invasion, impurities, and inflammatory reactions, a microorganism culturing production method has become recently a main one in which a molecular weight and productivity can be controlled, and a high quality of raw materials can be obtained. Especially, specific use of hyaluronic acid tends to be determined in accordance with the range of a molecular weight of hyaluronic acid adjusted and produced by the microorganism culturing method. An ultra-low molecular weight hyaluronic acid of 100,000 Da or less is mainly used for foods or cosmetics, while a low molecular weight hyaluronic acid with an average molecular weight of 1 million Da is utilized for developing an eye-drops raw material or its derivative. Hyaluronic acid with an average molecular weight of 3 million to 4 million Da is highly valuable when utilized as a raw material for a knee joint injection. In addition, its utilization as an ophthalmic surgery adjuvant has been increasing. As an ultra-high molecular weight material in the body, it has been highlighted for the purpose of a raw material for anti-adhesive agent.

Adhesion may be detected generally during a healing process from inflammation. Formation of adhesion in affected tissues occurs through clustering together or abundantly deposited fibrin when granulation tissue or scar forms. In general, an average of 67-93% among laparotomized patients develops adhesion. While spontaneously dissolving in some cases, adhesion maintains in most cases even after wound healing, causing various complications (See Eur. J. Surg. 1997, Suppl 577, 32-39). In order to prevent the formation of adhesion, wrapping around wound areas following surgery has been used, while anti-adhesion agents have been developed around the world, which block the formation of adhesion with surrounding tissues physically or chemically through their pharmacological functions or the like. Such anti-adhesion agents are produced by utilizing various high molecular materials among which arginic acid, CMC and hyaluronic acid are frequently used. As hyaluronic acid, cross-linked hyaluronic acid is utilized of which molecular weight, viscosity, elasticity and the like are enhanced from a low molecular weight hyaluronic acid. While anti-adhesion agents in the form of thin film product are commercially available, it is difficult to straighten the thin film and divide a single film product for the purpose of using in multiple areas. Hence, anti-adhesion agents in the form of gel have been developed which intend to be injected into surgical areas through a syringe. Due to a gel product's property of flowing down, a high molecular weight material is used for the gel product. In the case of hyaluronic acid, cross-linked hyaluronic acid is commonly used. Meanwhile, when cross-linked hyaluronic acid is utilized as a raw material for an anti-adhesion agent, what matters most is compounds used for cross-linking which may remain and cause an adverse effect in the body even after the degradation of hyaluronic acid as a biomaterial.

Regarding major patented domestic inventions in relation to an anti-adhesion composition comprising hyaluronic acid, Korean Registered Patent No. 10-1074467 discloses a method for preparing an anti-adhesion composition by mixing L-arginine and hyaluronic acid; Korean Registered Patent No. 10-0374666 and Laid-Open Korean Patent Application No. 10-2011-0114810 disclose a method for preparing hyaluronic acid in the form of gel by using sodium hyaluronate as a salt of hyaluronic acid, respectively. Laid-Open Korean Patent Application No. 10-2009-0012439 discloses the use of cross-linked hyaluronic acid prepared by mixing hyaluronic acid and a cross-linking agent. US Patent No. 6,630,167 discloses a preparation of an anti-adhesion composition by mixing a solution of hyaluronic acid and a solution of a cross-linking agent. US 2012/232261 A1 relates to a method for preparing low molecular weight hyaluronic acid from high molecular weight hyaluronic acid. According this application, the latter is produced using *Streptococcus* sp. ID91102 (KCTC11395BP) as the hyaluronic acid producing microorganism, which is cultured in a fermenter with glucose, casein peptone, magnesium sulfate, potassium dihydrogen phosphate, sodium chloride and glutamic acid and produces hyaluronic acid having an average molecular weight of 2,000,000 to 4,000,000 Da. EP 2 371 371 A2 relates to a composition for preventing tissue adhesion comprising hyaluronic acid and a polymer compound. More specifically, the hyaluronic acid has a molecular weight of 0.8 million to 3 million Da and is extracted from an animal tissue or prepared by a fermentation method, and a hydroxyethyl starch or chitooligosaccharide is used as the polymer compound.

As described above, it has not been found any disclosure in domestic and foreign prior arts in which non-cross linked or non-structurally transformed hyaluronic acid was used singularly. Further, both domestic and foreign prior arts generally expressed negative views on the single use of non-cross linked hyaluronic acid due to its easy degradation and thus short period of stay in the body.

Therefore, it is urgently needed to develop hyaluronic acid which is not cross-linked with long period of stay in the body and an anti-adhesion composition prepared by utilizing said hyaluronic acid.

### [Disclosure]

### [Technical Problem]

Accordingly, the inventors of the present invention have researched on a method of producing hyaluronic acid with long stay in the body and high efficacy for anti-adhesion to find that hyaluronic acid prepared by using *Streptococcus dysgalactiae* strain ID9103 is effective for inhibiting adhesion due to its low degradation rate in the body, leading to the completion of the present invention.

An object of the present invention is to provide a method for preparing hyaluronic acid with low degradation rate in the body, comprising a step of culturing *Streptococcus dysgalactiae* strain ID9103 (KCTC11818BP) in a medium comprising a carbon source and a nitrogen source as disclosed in claim 1.

Another object of the present invention is to provide a composition for use in inhibiting adhesion comprising non-cross linked hyaluronic acid prepared by said inventive method as an active ingredient.

Also disclosed is a method for inhibiting adhesion comprising a step of administering non-cross linked hyaluronic acid prepared by said inventive method to a subject in need thereof.

Further still another object of the present invention is to provide non-cross linked hyaluronic acid prepared by said inventive method and used for inhibiting adhesion.

### [Technical Solution]

To achieve the above-mentioned object, the present invention provides a method for preparing hyaluronic acid with a low degradation rate in the body, comprising a step of culturing *Streptococcus dysgalactiae* strain ID9103 (KCTC11818BP) in a medium comprising a carbon source and a nitrogen source, wherein the carbon source is maltose and the nitrogen source is casein enzymatic hydrolysate, and wherein the hyaluronic acid has a molecular weight of between 4 million Da and 10 million Da.

To achieve another above-mentioned object, the present invention provides a composition for use in inhibiting adhesion as disclosed in claim 5.

To achieve further still another above-mentioned object, the present invention provides non-cross linked hyaluronic acid as disclosed in claim 7.

Hereinafter, the present invention is described in detail.

The present invention provides a method for preparing hyaluronic acid with a low degradation rate in the body, comprising a step of culturing *Streptococcus dysgalactiae* strain ID9103 (KCTC11818BP) in a medium comprising a carbon source and a nitrogen source.

Preferably, the preparation method according to the present invention includes following steps:
(a) culturing *Streptococcus dysgalactiae* strain ID9103 (KCTC11818BP) in a medium comprising a carbon source and a nitrogen source; and
(b) collecting hyaluronic acid from the resulting culture in the medium of step (a).

The *Streptococcus dysgalactiae* strain ID9103 (accession number : KCTC-11818BP; Korean Patent Application No. 10-2011-0100364) is a microorganism which was isolated by separating hyaluronic acid-producing strains among microorganisms separated from cow feces, followed by causing mutation in the strains and then selecting a non-hemolytic strain that does not produce hyaluronidase.

The *Streptococcus dysgalactiae* strain ID9103 according to the present invention may be cultured by conventional methods for culturing microorganisms of genus *streptococcus*.

The culture medium comprises maltose as the carbon source and casein enzymatic hydrolysate as the nitrogen source. It may further comprise an amino acid or a metal ion.

The casein enzymatic hydrolysate is obtained by enzymatic decomposition of casein. For example, it may be tryptone, tryptone T, tryptone X, BBL biosate peptone, DIFCO casein digest, bacto casitone, BBL trypticase peptone, bacto tryptone, Bitec tryptone, NZ amine A, NZ amine AS, NZ amine EKC, NZ amine L concentration, NZ case, NZ case M, NZ case ME, NZ case plus, NZ case TT, pepticase, tryptone USP, pancreatic digest casein codex, pancreatic digest casein, enzymatic hydrolyzed casein kosher, or tryptone V.

The culture medium may further comprise an amino acid or a metal ion.

There is no limitation to the kind of the amino acid. Preferably, it may be selected from the group consisting of glutamine, lysine, cysteine, arginine, methionine, aspartic acid, glycine and a mixture thereof. More preferably, it may be arginine.

There is no limitation to the kind of the metal ion. Preferably, it may be selected from the group consisting of sodium, potassium, calcium, magnesium, iron, zinc, manganese and a mixture thereof. More preferably, it may be zinc.

More preferably, the culture medium according to the present invention may contain casein enzymatic hydrolysate as the nitrogen source, arginine as the amino acid, and zinc as the metal ion. While being cultured in the medium comprising casein enzymatic hydrolysate, arginine and zinc, the molecular weight of hyaluronic acid prepared by said microorganism according to the present invention may be modified to obtain hyaluronic acid with a molecular weight producing the most effective anti-adhesive function.

There is no limitation to the concentration of casein enzymatic hydrolysate, arginine and zinc, respectively. Preferably, the casein enzymatic hydrolysate is comprised at a concentration of 0.5%(w/v) to 3%(w/v), and arginine at a concentration of 0.01%(w/v) to 0.6%(w/v), and zinc at a concentration of 0.01%(w/v) to 0.1%(w/v).

There is no specific limitation to the culture method according to the present invention. For example, batch, fed-batch, or continuous culture methods may be used. In the present invention, a fed-batch culture method may be preferably used. In the fed-batch culturing, a culture medium being supplied to a fed-batch may comprise a nitrogen source, or both a nitrogen source and a carbon source, wherein the nitrogen source is casein enzymatic hydrolysate, and the carbon source is maltose.

The step of collecting hyaluronic acid from the preparation method according to the present invention may be conducted through known methods of isolating an active material from microorganism culture. Specifically, such known methods include bacteriostatic processes such as filtration, neutralizing processes, crystallization processes, and processes of removing and isolating impurities such as endotoxins, proteins, nucleic acids and metals (such as chromatography and centrifugation). Hyaluronic acid prepared by the method of the present invention is a high molecular weight hyaluronic acid having an average molecular weight of between 4 million Da and 10 million Da. More preferably, the high molecular weight hyaluronic acid may be selected from the group consisting of hyaluronic acids having an average molecular weight range of between 4 million Da and 6 million Da, and between 4 million Da and 8 million Da. Average molecular weight as described herein is weight average molecular weight.

Further, the preparation method according to the present invention may lead to the high yield production of high molecular weight hyaluronic acid with specified average molecular weight.

One example of the present application describes a preparation method according to the present invention in which a high molecular weight hyaluronic acid with its molecular weight ranging between 4,320,000 Da and 5,980,000 Da was obtained in a high yield of 8.07 g/L to 9.42 g/L (See Table 1).

The term "low degradation rate" as used herein means that hyaluronic acid is degraded or decomposed at a low rate upon being administered to the body of a subject.

Another example of the present application describes a comparative experiment on the degradation rate of hyaluronic acid prepared by the method according to the present invention in the body of a subject. In order to compare the degradation rate of hyaluronic acid in the body, hyaluronic acids having different molecular weights were treated with hyaluronidase which decomposes hyaluronic acid in the body, respectively, followed by measuring a viscosity value (cP) for each hyaluronic acid (3 million Da; 4 million Da; 6 million Da) over time. It was found that hyaluronic acids having the molecular weight of 4 million Da and 6 million Da respectively exhibited lower reduction rate in viscosity over time than one having the molecular weight of 3 million Da. Having low reduction rate in viscosity means that hyaluronic acid degrades or decomposes slowly in the body of a subject and thus is capable of maintaining its viscosity at a certain level. Thus, hyaluronic acid having the molecular weight of 4 million Da or more prepared by the method according to the present invention may possess a low degradation rate in the body of a subject. Preferably, hyaluronic acid having the molecular weight of 4 million Da to 6 million Da may be excellent for inhibiting adhesion.

Hyaluronic acid prepared by the method of the present invention includes hyaluronic acid having an average molecular weight of between 4 million Da and 10 million Da, more preferably between 4 million Da and 6 million Da. Hyaluronic acid having an average molecular weight of 3.5 million Da or less possesses high degradation rate in the body and thus lacks its effectiveness in inhibiting adhesion, while hyaluronic acid having an average molecular weight of 10 million Da or more is difficult to prepare.

As described above, hyaluronic acid prepared by the present invention has a low degradation rate in the body of a subject.

Further, an anti-adhesion composition comprising said hyaluronic acid as an active ingredient has low degradation rate in the body of a subject, resulting in an excellent effect in inhibiting adhesion.

The present invention provides a composition for use in inhibiting adhesion comprising, as an active ingredient, non-cross linked hyaluronic acid prepared by said method according to the present invention.

Also disclosed herein is a method for inhibiting adhesion comprising a step of administering, to a subject in need thereof, non-cross linked hyaluronic acid prepared by said method according to the present invention.

The disclosure also presents non-cross linked hyaluronic acid prepared by said method according to the present invention and used for inhibiting adhesion.

Furthermore, the present invention provides a composition for use in inhibiting adhesion comprising hyaluronic acid according to the present invention as an active ingredient. The composition according to the present invention is characterized by comprising hyaluronic acid according to the present invention. It may further comprise physiological saline solution, distilled water, sodium phosphate buffer solution and so on.

Hyaluronic acid according to the present invention has a lower degradation rate in the body of a subject than previously known hyaluronic acids, and thus even in its non-cross linked form possesses lubricative effect for inhibiting adhesion for a sufficient time, resulting in being used as a potent anti-adhesion agent.

The term "non-cross linked hyaluronic acid" as used herein refers to hyaluronic acid prepared by said preparation method according to the present invention which is not comprised of cross-linking chemical agents, chemical denaturants or cationic polymers forming a complex with hyaluronic acid.

The term "cross-linking chemical agents" as used herein refers to chemical compounds which react with hyaluronic acid to form a three dimensional network structure, and may be at least one selected from the group consisting of polyvalent epoxy compound such as polyglycidyl ether, divinyl sulfone, formaldehyde, phosphorous oxychloride, a mixture of carbodiimide compound and amino acid ester, and a mixture of carboiimide compound and dihydrazide compound.

The term "chemical denaturants" as used herein refers to chemical compounds which react with carboxyl, hydroxyl or acetamide substituents of hyaluronic acid to form covalent bonds, and may be at least one selected from the group consisting of a mixture of acetic anhydride and concentrated sulfuric acid, a mixture of anhydrous trifluoroacetic acid and organic acid, and alkyl iodine compound.

The term "cationic polymers forming a complex with hyaluronic acid" as used herein refers to polymer compounds which form a complex via ionic bonds between carboxyl substituents of hyaluronic acid and amino or imino substituents of the polymer compounds, and may be at least one selected from the group consisting of chitosan, polylysine, polyvinylpyridine, polyethyleneimine and polydimethylaminoethylmetacrylate.

The term "subject" as used herein refers to an animal, preferably a mammalian animal including humans, while including cells, tissues or organs originated from an animal. The "subject" may be a patient in need of treatment.

The term "administering" as used herein refers to, but not limited thereto, applying, distributing or attaching to a subject in need thereof a composition for inhibiting adhesion comprising hyaluronic acid according to the present invention as an active ingredient.

The composition according to the present invention may be administered to any body part of a subject, including visceral organs in abdominal and thoracic cavities, paratenons, cranial bones, nerves, bulbus oculi during laparotomy, gynecologic surgery and thoracotomy;tendons and ligaments during orthopedic surgery; and duramater during neurosurgery.

The composition according to the present invention may be used, but not limited thereto, in the form of gel, film or membrane.

### [Advantageous Effects]

As described above, the present invention provides a preparation method for hyaluronic acid possessing a low degradation rate in the body, and a composition for use in inhibiting adhesion comprising hyaluronic acid prepared by said preparation method. Hyaluronic acid prepared by the inventive method has an average molecular weight of between 4 million Da and 10 million Da with a potent effect in inhibiting adhesion due to its low degradation rate in the body. Therefore, since the composition comprising hyaluronic acid prepared by the inventive method possesses an excellent effect in inhibiting adhesion and utilizes non-cross linked hyaluronic acid, it is very effective in overcoming drawbacks of conventional hyaluronic acid and conventional anti-adhesion compositions containing cross-linking agents and chemical compounds.

### [Description of Drawings]

FIG. 1 is a picture showing the effect of hyaluronic acid in inhibiting adhesion based on its molecular weight in the open-cut abdomen of test animals.
FIG. 2 is a graph comparing the degradation rate of hyaluronic acid over time (Y axis: viscosity (cP); X axis: minutes (min)).

### [Mode for Invention]

Hereinafter, the present invention will be described in detail by referring to following examples.

However, the following examples are merely for illustrating the present invention, and are not intended to limit the scope of the present invention.

### <Example 1>

### Experiment on the effect of inhibiting adhesion based on the molecular weight of hyaluronic acid

SD rats (female, SPF, Orient Bio, Inc.) were anesthetized via inhalation and maintained under anesthesia throughout surgery. Their four limbs were tightly fixated on the operating table, followed by the removal of their abdominal hair with the aid of a razor. After disinfectants being applied, their abdomens were incised with operating scissors. Their appendixes were pulled out and rendered to be damaged by using coarse gauzes to the extent that the gauzes were smeared with bloodstains. The abdominal walls where the appendixes were located were also damaged in the same way as the appendixes, causing a condition where adhesion could occur between the appendixes and the internal abdominal walls. Test substances were administered and then the incision areas were sutured. After 2 weeks, laparotomy was performed to check the occurrence of adhesion. Said test substances included hyaluronic acids having an average molecular weight of 3million Da, 4 million Da and 6 million Da, respectively, dissolved in sodium phosphate buffer solution and maintained under an aseptic condition.

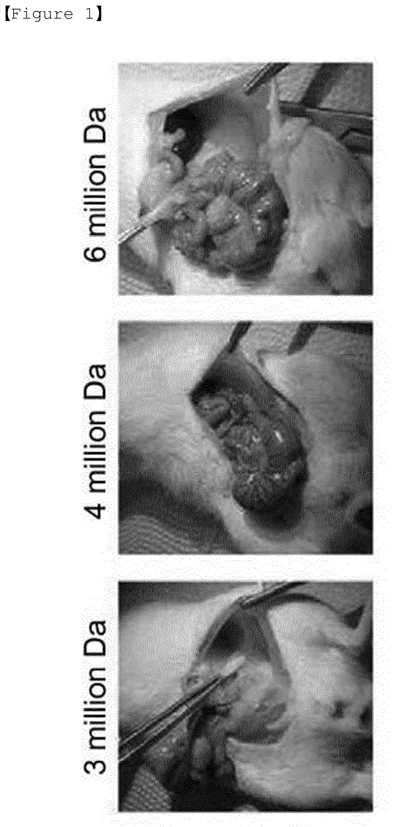

Adhesion between the abdominal organ and the abdominal walls was detected upon using hyaluronic acid of which molecular weight was 3 million Da. On the contrary, upon using hyaluronic acids of which molecular weights were 4 million Da or more, such an adhesion was not observed (See FIG. 1). Hence, it was confirmed that hyaluronic acid having a molecular weight of between 4 million Da and 6 million Da possesses an anti-adhesion effect.

### <Example 2>

### Basic culturing conditions for producing high molecular weight hyaluronic acid

4ml of *Streptococcus dysgalactiae strain* ID9103 culture solution stored in a -72°C refrigerator was rapidly thawed, smeared on 5.2% brain heart infusion solid medium, and cultured at 37°C for 24 hours. The grown colony was cut with an area of 1cm² and inoculated into 40ml of 3% Todd-Hewitt broth sterilized liquid medium (heart, infusion 0.31%, neopeptone 2%, dextropse 0.2%, NaCl 0.2%, Disodium phosphate 0.04%, sodium carbonate 0.25%; BD, US).

40ml of the liquid shake-cultured at 37°C and 150rpm was used as a primary seed culture solution. In a Logarithmic growth phase following culturing for 6 hours, the primary seed liquid was aseptically inoculated to three 3% Todd-Hewitt broth sterilized liquid media (40ml, pH 7.8). Under the culturing condition of 37°C and 150rpm, after aseptic culturing for 20 hours or more, the cultured medium was used as a secondary seed culture solution. Herein, the secondary seed culture solution should be maintained at pH of 6.4±0.2, and have OD (600) of 0.35±0.05. 80ml of the secondary seed culture solution was inoculated to a main culture medium, followed by culturing for 40 hours or more. The difference among hyaluronic acids in the productivity based on a medium composition was observed. Subsequently, the conditions for increasing the productivity of hyaluronic acid were determined. The culturing processesas described above were performed in the same manner in all Examples.

Tests of determining the main culture medium for optimally producing hyaluronic acid were conducted in a 7.5L fermentation bath under 3.5L culture solution condition. The medium composition was comprised of glucose 6%(w/v), yeast extract 0.5%(w/v), casein peptone 2%(w/v), glutamine 0.06%(w/v), sodium gluconate 0.1%(w/v), oxalic acid 0.02%(w/v), magnesium sulfate 0.15%(w/v), potassium phosphate dibasic 0.25%(w/v), sodium chloride 0.5%(w/v), sodium acetate 0.5%(w/v), ferric chloride 0.007 %(w/v), and ammonium molybdate 0.05 %(w/v). The tests were basically performed under the condition of pH 7.0, and 34°C.

In the present invention, the hyaluronic acid concentration in the culture solution was confirmed by both a carbazole method (T. Bitter, Anal. Biochem., 1962, 4, 330-334) and a turbidity analysis (S. Jung-Min, Carbohyd. Polym., 2009, 78, 633-634).

The average molecular weight of hyaluronic acid was obtained by a gel filtration chromatography method (Narlin B. Beaty et al,Anal. Biochem., 1985, 147, 387-395). In the analysis, the column was Toyo Soda TSK gelG6000PWXL, and the moving phase was comprised of 150mM NaCl, 3mM Na₂HPO₄(pH7.0), and 0.02% NaN₂. The detection was performed by a refractive index detector (Shodex; Showa Denko K.K. Japan), and the standard substance was prepared by polyethylene oxide at 2mg/ml concentration per molecular weight.

Hyaluronic acids prepared in accordance with the above described culturing conditions were detected to have a concentration of 7 g/L and a molecular weight of 3 million Da.

### <Example 3>

### Productivity and molecular weight of high molecular weight hyaluronic acid

It has been known that nitrogen source plays an important role in the metabolism of microorganisms and also affects the production of hyaluronic acid. Hence, the present inventors reasoned that changing the types of amino acids including a class of peptones used as a basic nitrogen source might contribute to the production of hyaluronic acid having a molecular weight of 4 million Da to 6 million Da as desired.

The present inventors confirmed that the concentration and the molecular weight of hyaluronic acid varied depending on the type of peptones. Among many different kinds of peptones, casein (enzymatic hydrolysate) used as the basic medium source lead to the best outcome, i.e. the production of hyaluronic acid having the concentration of 8 g/L or more and the molecular weight of 4,320,000 Da. Thus, all the experiments in following examples utilized casein (enzymatic hydrolysate).

The test group in which arginine was added instead of glutamine as a basic medium source resulted in an excellent outcome, i.e. hyaluronic acid having the concentration of 8.53 g/L or more and the molecular weight of about 4,830,000 Da was produced.

Carbon source also has been known to play an important role in the growth and metabolism of microorganisms, and has been utilized as a precursor of hyaluronic acids. Hence, the present inventors reasoned that changing the types of carbon source might affect the production of hyaluronic acid having a molecular weight of 4million Da to 6million Da as desired.

The test group in which maltose was added instead of glucose as a basic medium source resulted in an excellent outcome, i.e. hyaluronic acid having the concentration of 8.72 g/L or more and the molecular weight of about 5,520,000 Da was produced.

Metal ions have been known to play various roles inside the cells of microorganisms including the expression of DNAs and the activation of enzymes. Hence, under the assumption of their influence in the expression of DNAs and the activation of enzymes involved with the production of hyaluronic acid, various metal ions were tested.

The test group in which zinc was added resulted in an excellent outcome, i.e. hyaluronic acid having the concentration of 9.42 g/L or more and the molecular weight of about 5,980,000 Da was produced.

**[Table 1]**

| Difference in the concentration and the molecular weight of hyaluronic acid (HA) according to the type of peptones as a medium source | | |
|---|---|---|
| Type of peptones | Conc. of HA (g/L) | Molecular weight of HA (×1,000) |
| Casein peptone → Casein (enzymatic hydrolysate) | 8.07 | 4,321 |
| Glutamine → Arginine | 8.53 | 4,829 |
| glucose → Maltose | 8.72 | 5,523 |
| Zinc (added) | 9.42 | 5,981 |

### <Example 4>

### Comparative experiments on the degradation rate of hyaluronic acid in the body

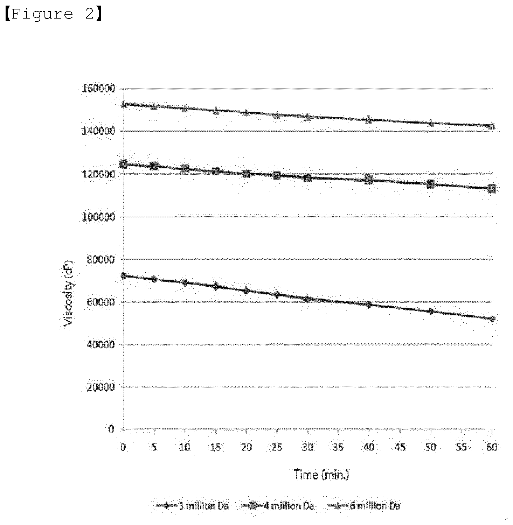

Comparative experiments on the degradation rate of hyaluronic acid having the molecular weight of 3 million Da, 4 million Da and 6million Da were respectively conducted by using hyaluronidase which degrades or decomposes hyaluronic acid in the body. Comparative experiments on degradation rate were performed to compare the difference in viscosity levels using the measured values (cP) between before and after the treatment with hyaluronidase. The measurement of viscosity was conducted using Brookfield Digital Viscometer LVDV-1+ (Brookfield, USA) with the setting of spindle 31, 0.3 RPM and 25°C. The results are shown in Table 2 and Figure 2.

**[Table 2]**

| Comparative experiments on the degradation rate of hyaluronic acid (HA) in the body according to its molecular weight | | | |
|---|---|---|---|
| Time after the addition of hyaluronidase (min.) | 3 million Da HA | 4 million Da HA | 6 million DaHA |
| 0 | 72,100 | 124,800 | 153,200 |
| 5 | 70,700 | 123,900 | 152,300 |
| 10 | 69,100 | 122,700 | 151,200 |
| 15 | 67,300 | 121,400 | 150,100 |
| 20 | 65,400 | 120,100 | 149,300 |
| 25 | 63,600 | 119,300 | 148,100 |
| 30 | 61,200 | 118,200 | 147,000 |
| 40 | 58,700 | 117,100 | 145,700 |
| 50 | 55,600 | 115,300 | 144,100 |
| 60 | 52,300 | 113,200 | 143,000 |
| Viscosity reduction rate | 27.5% | 9.3% | 6.7% |

As shown in Table 2 and Figure 2, hyaluronic acid of 3 million Da had its viscosity reduction rate of 27.5% which was quite high in comparison with those of hyaluronic acids of 4 million Da and 6 million Da, respectively. Hyaluronic acids of 4 million Da and 6 million Da, respectively, had about three or four times less viscosity reduction rate than hyaluronic acid of 3 million Da, confirming that hyaluronic acids of 4 million Da and 6 million Da possess lower degradation rate in the body and thus maintain their viscosity levels at a certain level. Thus, it was confirmed that hyaluronic acid having a molecular weight of 4 million Da or more prepared by the preparation method according to the present invention possesses a low degradation rate in the body, while, preferably, hyaluronic acid having a molecular weight of between 4 million Da and 6 million Da is excellent in inhibiting adhesion.

### [Industrial Applicability]

As described above, the present invention provides a preparation method for hyaluronic acid possessing a low degradation rate in the body, and a composition for inhibiting adhesion comprising hyaluronic acid prepared by said preparation method. Hyaluronic acid prepared by the inventive method is a high molecular weight hyaluronic acid having a molecular weight of between 4 million Da and 10 million Da with a potent effect in inhibiting adhesion due to its low degradation rate in the body. Therefore, since the composition for use in inhibiting adhesion comprising hyaluronic acid prepared by the inventive method possesses an excellent effect in inhibiting adhesion and utilizes non-cross linked hyaluronic acid, it is very effective in overcoming drawbacks of conventional hyaluronic acid and conventional anti-adhesion compositions containing cross-linking agents and chemical compounds.

## Claims

1. A method for preparing hyaluronic acid with a low degradation rate in the body of a subject, the method comprising a step of culturing *Streptococcus dysgalactiae* strain ID9103 (KCTC11818BP) in a medium comprising a carbon source and a nitrogen source,
wherein the carbon source is maltose and the nitrogen source is casein enzymatic hydrolysate,
and wherein the hyaluronic acid has a molecular weight of between 4 million Da and 10 million Da.

2. The method of claim 1, wherein the medium further comprises an amino acid or a metal ion.

3. The method of claim 2, wherein the amino acid is arginine and the metal ion is zinc.

4. The method of any of claims 1 to 3, wherein the hyaluronic acid has a molecular weight of between 4 million Da and 6 million Da.

5. A composition for use in inhibiting adhesion comprising as an active ingredient a non-cross linked hyaluronic acid prepared by a method of any one of claims 1 to 3.

6. A composition for use in inhibiting adhesion according to claim 5, wherein the composition is administered to a subject in need of inhibiting adhesion.

7. A non-cross linked hyaluronic acid prepared by a method of any one of claims 1 to 3 for use in inhibiting adhesion.

8. A non-cross linked hyaluronic acid for use in inhibiting adhesion according to claim 7, wherein the non-cross linked hyaluronic acid is administered to a subject in need of inhibiting adhesion.

## Patentansprüche

1. Verfahren zur Herstellung von Hyaluronsäure mit einer niedrigen Abbaurate im Körper eines Probanden, wobei das Verfahren einen Schritt des Kultivierens des *Streptococcus dysgalactiae*-Stammes ID9103 (KCTC11818BP) in einem Medium umfasst, das eine Kohlenstoffquelle und eine Stickstoffquelle umfasst,
wobei die Kohlenstoffquelle Maltose ist und die Stickstoffquelle enzymatisches Caseinhydrolysat ist,
und wobei die Hyaluronsäure ein Molekulargewicht zwischen 4 Millionen Da und 10 Millionen Da aufweist.

2. Verfahren nach Anspruch 1, wobei das Medium des Weiteren eine Aminosäure oder ein Metallion umfasst.

3. Verfahren nach Anspruch 2, wobei die Aminosäure Arginin ist und das Metallion Zink ist.

4. Verfahren nach einem der Ansprüche 1 bis 3, wobei die Hyaluronsäure ein Molekulargewicht zwischen 4 Millionen Da und 6 Millionen Da aufweist.

5. Zusammensetzung zur Verwendung bei der Hemmung von Verwachsung, umfassend als Wirkstoff eine nicht vernetzte Hyaluronsäure, hergestellt nach einem Verfahren nach einem der Ansprüche 1 bis 3.

6. Zusammensetzung zur Verwendung bei der Hemmung von Verwachsung nach Anspruch 5, worin die Zusammensetzung einem Subjekt verabreicht wird, bei dem Verwachsung gehemmt werden muss.

7. Nicht vernetzte Hyaluronsäure, hergestellt nach einem Verfahren nach einem der Ansprüche 1 bis 3, zur Verwendung bei der Hemmung von Verwachsung.

8. Nicht vernetzte Hyaluronsäure zur Verwendung bei der Hemmung von Verwachsung nach Anspruch 7, wobei die nicht vernetzte Hyaluronsäure einem Subjekt verabreicht wird, bei dem Verwachsung gehemmt werden muss.

## Revendications

1. Procédé de préparation d'acide hyaluronique à faible taux de dégradation dans le corps d'un sujet, le procédé comprenant une étape de culture de la souche ID9103 de *Streptococcus dysgalactiae* (KCTC11818BP) dans un milieu comprenant une source de carbone et une source d'azote,
dans laquelle la source de carbone est le maltose et la source d'azote est l'hydrolysat enzymatique de caséine,
et dans lequel l'acide hyaluronique a un poids moléculaire compris entre 4 millions et 10 millions Da.

2. Procédé selon la revendication 1, dans lequel le milieu comprend en outre un acide aminé ou un ion métallique.

3. Procédé selon la revendication 2, dans lequel l'acide aminé est l'arginine et l'ion métallique est le zinc.

4. Procédé selon l'une quelconque des revendications 1 à 3, dans lequel l'acide hyaluronique a un poids moléculaire compris entre 4 millions et 6 millions Da.

5. Composition destiné à être utilisé pour inhiber l'adhésion comprenant comme ingrédient actif un acide hyaluronique non réticulé préparé par un procédé selon l'une quelconque des revendications 1 à 3.

6. Composition destiné à être utilisé pour inhiber l'adhérence selon la revendication 5, dans laquelle la composition est administrée à un sujet ayant besoin d'inhiber l'adhésion.

7. Acide hyaluronique non réticulé préparé par un procédé selon l'une quelconque des revendications 1 à 3, destiné à être utilisé pour inhiber l'adhésion.

8. Acide hyaluronique non réticulé destiné à être utilisé dans l'inhibition de l'adhésion selon la revendication 7, dans lequel l'acide hyaluronique non réticulé est administré à un sujet ayant besoin d'inhiber l'adhérence.
